# EUROPÄISCHE PATENTSCHRIFT

(11) **EP 2 091 934 B1**
(45) Veröffentlichungstag und Bekanntmachung des Hinweises auf die Patenterteilung: **24.04.2013**
(21) Anmeldenummer: 07822235.3
(22) Anmeldetag: 06.11.2007
(51) Int. Cl.: C07D 317/20, C07D 319/06, C07D 321/08, C07C 319/20, C07C 323/52, C07C 323/60, A23K 1/16

(54) **KETOMETHIONINKETALE UND DEREN DERIVATE**
KETOMETHIONINE KETALS AND THEIR DERIVATIVES
CÉTOMÉTHIONINE CÉTALS ET LEUR DÉRIVÉS

(30) Priorität: 24.11.2006 DE 102006055470
(43) Veröffentlichungstag der Anmeldung: 26.08.2009
(73) Patentinhaber: Evonik Degussa GmbH, 45128 Essen (DE)
(72) Erfinder: KOBLER, Christoph, 63755 Alzenau (DE); HATELEY, Martin, 81825 München (DE); ROTH, Philipp, 63456 Hanau (DE); JÄGER, Barbara, 63533 Mainhausen (DE); PETER, Rainer, 63829 Krombach (DE); WECKBECKER, Christoph, 63584 Gründau-Lieblos (DE); HUTHMACHER, Klaus, 63571 Gelnhausen (DE)
(86) Internationale Anmeldenummer: PCT/EP2007/061908
(87) Internationale Veröffentlichungsnummer: WO 2008/061871

(56) Entgegenhaltungen:
- EP-A- 0 400 499
- WO-A-2006/072711
- US-A1- 2005 187 293

## Beschreibung

### Einleitung

Die vorliegende Erfindung betrifft Ketomethioninketale bzw. -halbketale und deren Derivate sowie deren Herstellung und deren Verwendung als Futtermitteladditive, insbesondere für die Ernährung von Wiederkäuern.

### Stand der Technik

Aminosäuren wie Methionin, Lysin oder Threonin sind als Futtermitteladditive wichtige Bestandteile der Tierernährung. Sie ermöglichen ein schnelleres Wachstum sowie eine effizientere Verwertung des Futters. Dies stellt meist einen deutlichen wirtschaftlichen Vorteil dar. Die Märkte für Futtermitteladditive sind von großer industrieller und wirtschaftlicher Bedeutung. Zudem sind sie starke Wachstumsmärkte, was nicht zuletzt auf die steigende Bedeutung von Ländern wie beispielsweise China und Indien zurückzuführen ist.

Aus WO 2004008874 ist unter anderem bekannt, dass Methionin für vielen Tierarten, darunter auch Wiederkäuer, die erste limitierende Aminosäure darstellt. So ist beispielsweise bei Milchkühen die effiziente Milchproduktion hinsichtlich der Menge und Qualität sehr stark von einer ausreichenden Zufuhr von Methionin abhängig. Der Methioninbedarf von Hochleistungsmilchkühen kann dabei nicht durch das im Pansen gebildete Mikrobeneiweiß bzw. durch im Pansen nicht abgebautes Eiweiß aus dem Futter gedeckt werden (Graulet et al., J. Animal and Feed Sciences (2004), 269). Es ist daher vorteilhaft, Methionin dem Futter zu supplementieren, um die Wirtschaftlichkeit der Milchproduktion und die Qualität der Milch zu erhöhen.

Bei monogastrischen Tieren wie z.B. Geflügel und Schweinen wird üblicherweise Methionin und das Methionin-Hydroxy-Analog (MHA), welches auch als Hydroxymethylthiobuttersäure (HMB) bezeichnet wird, als Futtermitteladditiv verwendet. Dadurch wird die verfügbare Menge an L-Methionin im Organismus erhöht, die dann dem Tier zum Wachstum zur Verfügung stehen kann.

Im Gegensatz dazu ist die Supplementierung des Futters mit Methionin bei Wiederkäuern nicht effektiv, da die Hauptmenge im Pansen (Rumen) der Wiederkäuer durch Mikroben abgebaut wird. Aufgrund dieses Abbaus gelangt daher nur ein Bruchteil des supplementierten Methionins in den Dünndarm des Tiers, wo im Allgemeinen die Absorption des Methionins ins Blut erfolgt.

In WO 99/04647 wird die Verwendung von MHA für Wiederkäuer beschrieben. Darin wird behauptet, dass MHA nur zum Teil im Pansen abgebaut wird und daher mindestens 20-40% des supplementierten MHAs nach Absorption im Dünndarm in den Stoffwechsel gelangen können. In zahlreichen anderen Publikationen wird dagegen die Wirkungsweise von MHA beim Wiederkäuer unterschiedlich diskutiert. So wird beispielsweise in WO 200028835 beschrieben, dass MHA nur dann den Pansen erfolgreich passieren und schlussendlich zur Absorption in den Dünndarm gelangen kann, wenn MHA in sehr großen Mengen von 60-120g/Tag/Tier verabreicht wird. Dadurch ist jedoch eine Wirtschaftlichkeit nicht mehr gegeben.

Damit dem Wiederkäuer Methioninprodukte wie D,L-Methionin bzw. *rac*-MHA mit hoher Effizienz zur Verfügung stehen, muss eine vor dem Pansenabbau geschützte Form eingesetzt werden. Die Herausforderung ist hierbei, ein geeignetes Methioninprodukt aufzufinden, das dem Methionin eine möglichst hohe Pansenstabilität verleiht und trotzdem eine hohe und effiziente Absorption des Methionins im Darm gewährleistet. Dabei gibt es mehrere Möglichkeiten, dem

D,L-Methionin oder rac-MHA diese Eigenschaften zu verleihen:

### a)Physikalischer Schutz:

Durch Anbringung einer geeigneten Schutzschicht bzw. Verteilung des Methionins in einer Schutzmatrix kann eine hohe Pansenstabiliät erreicht werden. Dadurch kann das Methionin den Pansen praktisch ohne Verlust passieren. Im weiteren Verlauf wird die Schutzschicht dann z. B. im Labmagen durch saure Hydrolyse geöffnet oder entfernt und das freiwerdende Methionin kann dann im Dünndarm vom Tier absorbiert werden. Die Schutzschicht bzw. -matrix kann aus einer Kombination von mehreren Substanzen wie z.B. Lipiden, anorganische Materialien und Kohlenhydraten bestehen. Folgende Produktformen sind kommerziell erhältlich:
i) Met-Plus™ von Nisso America ist ein lipidgeschütztes Methionin mit einem D,L-Methioningehalt von 65%. Die Schutzmatrix besteht aus den Calciumsalzen langkettiger Fettsäuren wie z.B. Laurinsäure. Als Konservierungsstoff dient butyliertes Hydroxytoluol.
ii) Mepron® M85 von Degussa AG ist ein kohlenhydratgeschütztes Methionin, das einen Kern aus D,L-Methionin, Stärke und Stearinsäure besitzt.
   Als Schutzschicht wird Ethylcellulose verwendet. Das Produkt hat einen Gehalt von 85% D,L-Methionin.
iii) Smartamine™ M von Adisseo ist ein polymergeschütztes Methionin. Die Pellets enthalten neben Stearinsäure mind. 70% D,L-Methionin. Die Schutzschicht enthält Vinylpyridin-Styrol-Copolymer.

Obwohl der physikalische Schutz den mikrobiellen Abbau des Methionins im Pansen verhindert und dadurch die Zufuhr und Verwertung von Methionin im Tier erhöht werden kann, gibt es einige gravierende Nachteile.

Die Herstellung bzw. die Beschichtung von Methionin stellt meist ein technisch kompliziertes und aufwendiges Verfahren dar und ist daher teuer. Zudem kann die oberflächliche Beschichtung der fertigen Pellets leicht durch mechanische Belastung und Abrieb während der Futterverarbeitung beschädigt werden, was zur Verminderung bzw. bis zum vollständigen Verlust des Schutzes führen kann. Deshalb ist es auch nicht möglich, die geschützten Methioninpellets in ein größeres Mischfutterpellet zu verarbeiten und neu zu pelletieren, da dadurch wiederum die schützende Schicht durch die mechanische Beanspruchung aufbrechen würde. Dies schränkt die Verwendung solcher Produkte stark ein, da die Mischfutterpelletierung eine weit verbreitete Methode der Futterverarbeitung darstellt.

### b) Chemischer Schutz:

Eine erhöhte Pansenstabilität von Methionin kann neben den rein physikalischen Schutzmöglichkeiten auch durch Modifikation der chemischen Struktur, beispielsweise durch Veresterung der Carbonsäuregruppe, erreicht werden. Zurzeit sind folgende Produkte kommerziell erhältlich oder in der Literatur beschrieben:
i) Methioninester wie z.B. D,L-tert-Butylmethionin: Die Ester wurden getestet und zeigten nur einen moderate Pansenstabilität (Loerch und Oke; "Rumen Protected Amino Acids in Ruminant Nutrition" in "Absorption and Utilization of Amino Acids" Vol. 3, 1989, 187-200, CRC Press Boca Raton, Florida). Für D,L-tert-Butylmethionin wurde dagegen in WO 0028835 eine Biowertigkeit von 80% veröffentlicht.
ii) Metasmart™ von Adisseo ist der racemische iso-Propylester von MHA (HMBi). Diese Verbindung wird auch unter dem Trademark "Sequent" von der amerikanischen Firma Novus vermarktet. In WO 00/28835 wurde eine Biowertigkeit von mindestens 50% für HMBi bei Wiederkäuern veröffentlicht. Dabei spielt vor allem die überraschend schnelle Absorption des hydrophoben HMBi's über die Pansenwand eine entscheidende Rolle. Der Ester kann dann im Blut zu MHA hydrolysiert und nach Oxidation und anschließender Transaminierung zum L-Methionin umgewandelt werden. Im Patent EP 1358805 wurde eine vergleichbare Biowertigkeit für HMBi publiziert. Bei diesen Untersuchungen war HMBi auf einem porösen Träger aufgebracht. In einer weiteren Veröffentlichung wurde von der Europäischen Kommission berichtet, dass wiederum ca. 50% HMBi über die Pansenwand absorbiert werden (European Commission: Report of the Scientific Committee on Animal Nutrition on the Use of HMBi; 25 April 2003). Graulet et al. veröffentlichte 2004 im Journal of Animal and Feed Science (269), dass durch die lipophilen Eigenschaften der iso-Propylgruppe von HMBi eine bessere Diffusion über die Pansenwand ermöglicht wird.
   Zur Herstellung von HMBi sind zwei verschiedene Verfahren veröffentlicht worden. So kann HMBi entweder direkt in einer Stufe aus dem entsprechenden Cyanhydrin (WO 00-59877) dargestellt werden. Die Veresterung zum iso-Propylester erfolgt dabei *in situ*, ohne zuvor MHA isolieren zu müssen. Ein anderes Verfahren verestert dagegen reines MHA mit *iso*Propanol (WO 01-58864 und WO 01-56980). In beiden Fällen wird zur Synthese Blausäure verwendet, die teuer ist und zudem ein großes Gefahrenpotenzial in sich birgt.
iii) Ketomethionin und seine Carbonsäurederivate: Die Anwendung dieser Verbindungsklasse insbesondere von Ketomethionin selbst als Futtermitteladditive wurde erst vor kurzem in Anmeldung WO 2006-072711 beschrieben. Dort wurde auch ein technisches Verfahren zur Herstellung von Ketomethionin und seiner Carbonsäurederivate beschrieben. Ketomethionin stellt die direkte Vorstufe von Methionin dar und kann im Organismus in einem Schritt mittels Transaminierung leicht zu L-Methionin umgesetzt werden. Im Vergleich dazu besitzen sowohl MHA als auch HMBi den Nachteil, dass sie zwei bzw. drei Schritte zur Umwandlung zu L-Methionin im Organismus benötigen. So muss HMBi zuerst zu freiem MHA hydrolysiert und anschließend mit Hilfe einer Oxidase zum Ketomethionin oxidiert werden. Erst dann kann wiederum das Ketomethionin direkt zu L-Methionin reduktiv aminiert werden [Baker; "Utilization of Precursors for L-Amino Acids" in "Amino Acids in Farm Animal Nutrition" (D'Mello, J.P.F., ed.), 1994, 37-64. CAB Intl., Wallingford, Oxon, UK.]

Das freie Ketomethionin als α-Ketocarbonsäure und seine Salze wie z.B. das Natrium- oder Calciumsalz sind bereits seit längerer Zeit literaturbekannte Verbindungen und wurden sowohl biochemisch als auch chemisch hergestellt. Meister erhielt beispielsweise das Natriumsalz von α-Ketomethionin in einer Ausbeute von 77% durch die L-Aminooxidasen katalysierte Oxidation von Methionin (Meister, J. Biol. Chem. 1952, 197, 309). Zuvor zeigte Waelsch et al., dass, die in Leber enthaltenen Aminooxidasen, Methionin zu α-Ketomethionin umwandeln können (Waelsch et al., J. Am. Chem. Soc. 1938, 61, 2252). Mosbach et al. beschrieb ebenfalls die Herstellung von Ketomethionin durch die L-Aminooxidasen katalytisierte Oxidation von Methionin. Dabei wurden immobilisierte Providencia sp. PCM 1298 Zellen verwendet (Mosbach et al., Enzyme Microb. Technol. 1982, 4, 409).

Als weitere Synthesemöglichkeit veröffentlichte Sakurai et al. 1957 die erste chemische Syntheseroute zur Darstellung von α-Ketomethionin. Dabei wurde als Schlüsselschritt Methyl-α-methoxalyl-γ-methylmercapto-propionat mit verdünnter Salzsäure zu Ketomethionin hydrolysiert (Sakurai et al., J. of Biochemistry 1957, 44, 9, 557). Yamada et al. publizierte fast zeitgleich dieselbe Syntheseroute, nachdem erste Versuche zur Darstellung von α-Ketomethionin über einen intermediär gebildeten α-Oximoester lediglich niedrige Ausbeute lieferten (Chibata et al., Bull. Agr. Chem. Soc. Japan 1957, 21, 6, 336).

Die Biowertigkeit des Natriumsalzes von α-Ketomethionin wurde erstmals 1977 in Fütterungsversuchen bei Nagetieren und Geflügel bestimmt und liegt deutlich über der von MHA (Baker und Harter, Proceedings for the Society for Experimental Biology and Medicine 1977, 156, 2001). Bei Wiederkäuern wird α-Ketomethionin bzw. dessen Salze jedoch im Pansen abgebaut und bietet daher keinerlei Vorteile gegenüber HMB oder Methionin. Als freie α-Ketocarbonsäure hat α-Ketomethionin zudem noch den Nachteil, dass es in kürzester Zeit dimerisiert und anschließend irreversibel cyclisiert und daher als biowertiges Monomer nicht stabil ist, und sich daher einer direkten Verwendung als Futtermitteladditiv entzieht.

### Aufgabe der Erfindung

Vor dem Hintergrund der Nachteile des Standes der Technik war es die Aufgabe, ein pansenstabiles, chemisch geschütztes Methioninprodukt für Wiederkäuer, insbesondere für Milchkühe bereitzustellen, das den Tieren als effiziente Methioninquelle dienen kann und die Nachteile der bekannten Produkte nicht oder in verringertem Umfang aufweist. Dies hätte den Vorteil, dass sich damit die Milchproduktion und -qualität erhöhen lassen könnte, was zu einer deutlichen Steigerung der Wirtschaftlichkeit führen würde.

Eine weitere Aufgabe war es, ein Futtermittel bzw. einen Futtermittelzusatzstoff mit sehr hoher Biowertigkeit aufzufinden, das gute Handhabbarkeit und Lagerfähigkeit sowie Stabilität unter den üblichen Bedingungen der Mischfutterverarbeitung, insbesondere der Pelletierung aufweisen sollte. Das Produkt sollte darüber hinaus möglichst generell als Futtermittelzusatzstoff in der Tierernährung einsetzbar sein.

### Beschreibung der Erfindung

Gelöst werden diese sowie weitere nicht explizit genannte Aufgaben, die jedoch aus den hierin diskutierten Zusammenhängen ohne weiteres ableitbar oder erschließbar sind durch die erfindungsgemäßen Ketomethioninketale und deren Derivate gemäß Formel I, insbesondere deren Verwendung als Futtermittel, vorzugsweise für Wiederkäuer. Damit werden sowohl die Nachteile der "physikalisch geschützten" Methioninvarianten wie z.B. Smartamine als auch die Nachteile der "chemisch geschützten" Varianten wie z.B. HMBi überwunden.

Gegenstand der vorliegenden Erfindung ist eine chemische Verbindung der allgemeinen Formel I 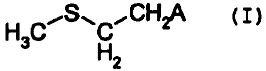 wobei A = 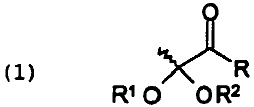 und R = OR', ist, wobei R¹, R² und R' gleich oder verschieden ist und jeweils ein verzweigtes oder geradkettiges C₁-C₁₈-Alkyl- bzw.
C₃-C₁₈-Cycloalkyl-,
Allyl-,
Benzyl-,
Phenyl- oder
C₁-C₁₈-Alkyloxymethyl-, vorzugsweise C₂H₅OCH₂
C₂-C₆-Hydroxyalkyl-, vorzugsweise HOC₂H₄-,
C₃-C₆-Dihydroxyalkyl-, vorzugsweise (HO)₂C₃H₅-, oder ein C₃-C₁₂-Zuckerrest, bei dem eine OH-Gruppe des Zuckers jeweils vom Ketal-O-Atom oder vom Carbonsäure- O-Atom ersetzt ist,
oder A = 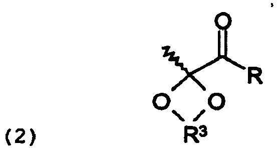 und R = OR' ist,
wobei R' jeweils ein verzweigtes oder geradkettiges C₁-C₁₈-Alkyl- bzw.
C₃-C₁₈-Cycloalkyl-,
Allyl-,
Benzyl-,
Phenyl- oder
C₁-C₁₈-Alkyloxymethyl-, vorzugsweise C₂H₅OCH₂-,
C₂-C₆-Hydroxyalkyl-, vorzugsweise HOC₂H₄-,
C₃-C₆-Dihydroxyalkyl-, vorzugsweise (HO)₂C₃H₅-, oder ein C₃-C₁₂-Zuckerrest, bei dem eine OH-Gruppe des Zuckers vom Carbonsäure-O-Atom ersetzt ist
und R³ eine C₂-C₄-Alkylenbrücke darstellt, vorzugsweise C₂H₄, oder einen C₃-C₁₂-Zuckerrest, bei dem zwei OH-Gruppen des Zuckers von den beiden Ketal-O-Atomen ersetzt sind,
oder A = 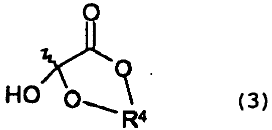 und R⁴ ein C₃-C₆-Alkylenrest, der ggf. Hydroxysubstituiert ist, vorzugsweise -CH₂-C(CH₃)₂-CH₂- und -CH₂-C(CH₂OH)₂-CH₂- ist, oder ein C₃-C₁₂-Zuckerrest, bei dem eine OH-Gruppe des Zuckers vom Ketal-O-Atom und eine vom Carbonsäure-O-Atom ersetzt ist,
oder A = 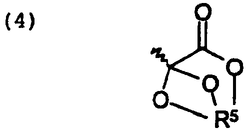 und R⁵ ein C₃-C₆-Alkylenrest, der ggf. hydroxysubstituiert ist, vorzugsweise -CH₂-CH(-)-CH₂-, ist, oder ein C₃-C₁₂-Zuckerrest, bei dem drei OH-Gruppen des Zuckers von beiden Ketal-O-Atomen und vom Carbonsäure-O-Atom ersetzt sind.

Gegenstand der Beschreibung (aber nicht der Erfindung) ist eine chemische Verbindung der allgemeinen Formel I, wobei
A= 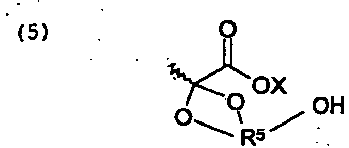 und R⁵ ein C₃-C₆-Alkylenrest, der ggf. hydroxysubstituiert ist, vorzugsweise -CH₂-CH(-)-CH₂-, oder ein C₃-C₁₂-Zuckerrest, bei dem zwei OH-Gruppen des Zuckers von den beiden Ketal-O-Atomen ersetzt sind, und wobei X=H oder M ist und M die oben angegebene Bedeutung hat.

Teil der Beschreibung (aber nicht der Erfindung) ist auch eine Verbindung der Formel I, 2, bei der R = Hydroxy und R³ = C₂H₄ ist:

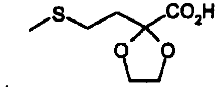

Diese ist leicht herstellbar durch Ketalisierung von Ketomethionin mit stöchiometrischen Mengen Ethylenglykol in Gegenwart saurer Katalysatoren oder mit überstöchiometrischen Mengen Ethylenglykol und anschließende Verseifung des intermediär gebildeten Ketomethioninethylenketalethylenglykolesters mit Alkali und nachfolgender Neutralisation (vgl. Beispiel 2).

Erfindungsgemäß bevorzugt ist eine Verbindung gemäß Formel I,2, bei der R = OR' und R'= C₁-C₁₈-Alkyl und R³ = C₂H₄, ist. Diese Verbindung kann hergestellt werden durch Veresterung von Ketomethionin mit einem entsprechenden C₁-C₁₈-Alkohol in Gegenwart saurer Katalysatoren und vorzugsweise anschließender Ketalisierung mit Ethylenglykol.

Besonders bevorzugt ist dabei eine Verbindung gemäß Formel I,2, bei der R' = C₁-C₄-Alkyl ist.

Weiterhin bevorzugt ist eine Verbindung gemäß Formel I,2, bei der R = OR' und R'= Hydroxyethoxy und R³ = C₂H₄ ist:

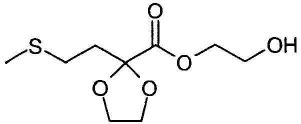

Darüber hinaus bevorzugt ist eine Verbindung gemäß Formel 1,3, bei der R⁴ ein C₅H₁₀-Rest oder ein C₅H₁₀O₂-Rest ist.

Besonders bevorzugt ist dabei eine Verbindung, gekennzeichnet durch die Formel 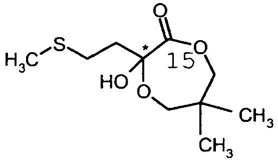 welche aus Ketomethionin und Neopentylidenglykol in Gegenwart saurer Katalysatoren hergestellt werden kann.

Ebenso bevorzugt ist eine analoge Verbindung der Formel 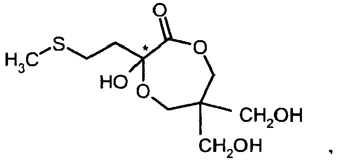 welche aus Ketomethionin und Pentaerythrit in Gegenwart saurer Katalysatoren hergestellt werden kann.

Weiterhin bevorzugt ist eine Verbindung gemäß Formel 1,4, bei der R⁵ ein C₃H₅-Rest ist.

Besonders bevorzugt ist dabei eine Verbindung gekennzeichnet durch die Formel (1,2-Ketal) 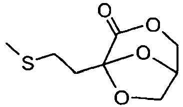 oder die isomere Formel (1,3-Ketal)

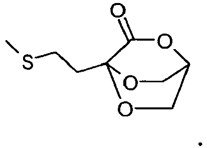

Beide Verbindungen - das 1,2-Ketal und das 1,3-Ketal - können aus Ketomethionin und Glycerin in Gegenwart saurer Katalysatoren, vorzugsweise unter wasserentziehenden Bedingungen hergestellt werden.

Alle Verbindungen der allgemeinen Formel I eignen sich erfindungsgemäß hervorragend zur Verwendung in der Tierernährung, vorzugsweise zur Ernährung von Nutztieren.

Dabei bevorzugt ist die Verwendung zur Ernährung von Wiederkäuern, insbesondere von Milchkühen.

Zu diesem Zweck können die erfindungsgemäßen Verbindungen zur Herstellung von Futtermitteln verwendet werden.

Dabei wiederum bevorzugt ist die Verwendung zur Herstellung von Futtermitteln für Wiederkäuer.

Ebenso Gegenstand der vorliegenden Erfindung sind Futtermittelzubereitungen, enthaltend mindestens eine der vorstehend genannten erfindungsgemäßen Verbindungen, vorzugsweise zur Ernährung von Wiederkäuern.

Demgemäß können Mischungen der erfindungsgemäßen Verbindungen mit gängigen Futtermitteln hergestellt werden. Dazu werden die erfindungsgemäßen Verbindungen in geeigneten Mengen in handelsübliche Futterarten, wie Mineralfutter, organische Futter (z.B. Sojaschrot) oder Milchleistungsfutter eingemischt.

Geeignete Mengen sind in der Regel Anteile von 0,1% bis 5% an Methioninäquivalenten in Form der erfindungsgemäßen Verbindungen, wobei die Anteile je nach Futterart verschieden sind. Milchleistungsfutter wird vorzugsweise mit < 0.5% an Methioninäquivalenten gemischt, Mineralfutter mit bis zu 5% und organische Futter im Bereich von 0,5 bis 3%, vorzugsweise bis 1%. Ein Methioninäquivalent ist in diesem Zusammenhang der Gewichtsanteil an erfindungsgemäßer Verbindung, der der gleichen Menge an Methionin auf molarer Basis entspricht.

Ein weiterer Gegenstand der vorliegenden Erfindung ist ein Verfahren zur Herstellung von Carbonsäure- oder Esterverbindungen der allgemeinen Formel I, 1-4, dadurch gekennzeichnet, dass man Ketomethionin mit einem entsprechenden einwertigen, zweiwertigen, dreiwertigen Alkohol oder einem C₃-C₁₂-Zucker in Gegenwart saurer Katalysatoren zum Esterprodukt der Formel 1 - 2, mit R = OR' ; oder zum Esterprodukt 3-4 umsetzt.

Entsprechend der genannten bevorzugten Verbindungen wird dabei ein Verfahren bevorzugt, bei dem als Alkohol ein verzweigter oder geradekettiger C₁-C₁₈-Alkylalkohol bzw.C₃-C₁₉-Cycloalkylalkohol, Allylalkohol, Benzylalkohol, Phenylalkohol, C₂-C₆-Hydroxyalkylalkohol, vorzugsweise HOC₂H₄OH, C₃-C₆-Dihydroxyalkylalkohol, vorzugsweise Glycerin, oder einen C₃-C₁₂-Zucker, bevorzugt Glycerinaldehyd, Dihydroxyaceton, Glucose, Fructose oder Saccharose eingesetzt werden.

Dabei kann insbesondere durch geeignete Auswahl der Menge der Moläquivalente des zur Ketalisierung benötigten Alkohols bzw. Zuckers die Bildung des gewünschten Produktes beeinflusst werden. Bei Verwendung von einem Moläquivalent C₁-C₁₈-Alkylalkohol bildet sich bevorzugt der nichterfindungsgemäße entsprechende Ester, bei Verwendung von zwei Moläquivalenten bildet sich bevorzugt das Carbonsäureketal der Formel I, 1 mit R = OH und bei Verwendung von drei Moläquivalenten bevorzugt die entsprechenden Esterketale der Formel I, 1 mit R = OR'. Entsprechend wird bei Einsatz von einem halben Moläquivalent C₂-C₆-Hydroxyalkylalkohol - wie z.B. HOC₂H₄OH -, entsprechend zwei Hydroxyäquivalenten das entsprechende Carbonsäureketal der Formel I, 2 mit R = OH und R³ = C₂H₄ gebildet.

Die Herstellung der Carbonsäure- oder Esterverbindungen kann vorteilhaft in Gegenwart eines Lösungsmittels durchgeführt werden. Geeignete Lösungsmittel sind beispielsweise aromatische Kohlenwasserstoffe wie Benzol oder Toluol sowie chlorierte Kohlenwasserstoffe wie Methylenchlorid oder Chloroform und Alkohole.

Im Interesse einer möglichst hohen Ausbeute an gewünschten Kondensationsprodukten wird dabei ein Verfahren bevorzugt, bei dem während der Reaktion entstehendes Wasser aus dem Gleichgewicht entfernt wird. Dies kann sowohl destillativ, vorzugsweise durch Verwendung eines Lösungsmittels und/oder Schleppmittels wie beispielsweise Toluol erreicht werden. Vorteilhaft dabei ist, wenn das Lösemittel gleichzeitig Schleppmittel und ggf. noch als Reaktant fungiert. Auch können dabei die zur Veresterung bzw. Ketalisierung eingesetzten Alkohole zusätzlich als Lösungsmittel und/oder Schleppmittel verwendet werden.

Bei Verwendung als Schleppmittel sollte das Lösungsmittel einen geeigneten Siedepunkt haben. Dieser sollte in der Regel 120°C nicht überschreiten. Für diesen Zweck geeignete Alkohole sind z.B. C₁-C₄-Alkylalkohole, also Methanol, Ethanol, 1- oder 2-Propanol und 1- oder 2-Butanol, Isobutanol, *tert*-Butanol.

Eine andere Möglichkeit zur Wasserentfernung besteht in der Verwendung von Wasserentziehenden Mitteln beispielsweise von Orthoestern, wie zum Beispiel Orthoessigsäuretrimethylester oder -triethylester. Die dabei als Koppelprodukte entstehenden Essigsäuremethyl- bzw. - ethylester können anschließend leicht destillativ aus dem Reaktionsgemisch entfernt und weiterverwendet werden.

Dabei können die nicht erfindungsgemäßen oder Carbonsäuresalze der allgemeinen Formel I, 1-2 oder 5, bei denen der Rest R = OH oder OM und der Rest X = H oder M ist, bevorzugt so hergestellt werden, indem ein Carbonsäureesterprodukt der Formel I, 1 oder 2 mit R = OR', oder der Formel I, 4 durch Verseifung mit einem Alkali- oder Erdalkalihydroxid oder einem ein- bzw. zweiwertigen Übergangsmetallhydroxid in das entsprechende Carbonsäuresalz der Formel I, 1 oder 2 mit R = OM bzw. der Formel 5 mit X = M überführt wird und ggf. daraus die freie Carbonsäure mit R = OH bzw. X= H mit Hilfe einer Mineralsäure freigesetzt wird. Geeignete Mineralsäuren sind dabei insbesondere Schwefelsäure, Salzsäure und Phosphorsäure.

Gegenstand der Beschreibung (aber nicht der Erfindung) ist auch ein Verfahren zur Herstellung von Carbonsäuresalzen der allgemeinen Formel I, 1- 2 oder 5, wobei der Rest R = OM bzw. der Rest X = M ist, das dadurch gekennzeichnet ist, dass ein Säureprodukt der Formel I, 1 -2 mit R = OH bzw. ein Säureprodukt der Formel I, 5 mit X= H mit einem Alkali- oder Erdalkali- oder einem ein- bzw. zweiwertigen Übergangsmetallhydroxid oder - carbonat zur entsprechenden Carbonsäure neutralisiert wird.

Zur Herstellung der oben genannten Carbonsäuresalze durch Verseifung oder Neutralisation werden dabei als Hydroxide NaOH, KOH, Mg (OH)₂, Ca (OH)₂, Zn(OH)₂ oder Mn(OH)₂ und als Carbonate Na₂CO₃, K₂CO₃, MgCO₃, CaCO₃, ZnCO₃ oder MnCO₃ bevorzugt verwendet.

Gegenstand der Beschreibung (aber nicht der Erfindung) ist auch ein Verfahren zur Herstellung von Carbonsäureamiden der allgemeinen Formel I, 1-2 mit R = NH₂, NHR', NR'R" , dadurch gekennzeichnet, dass ein Esterprodukt der Formel I, 1 -2, mit R = OR', durch Umsetzung mit einer N-haltigen Base der Formel NH₃, NH₂R' oder NHR'R" in das entsprechende Amid überführt wird.

Gegenstand der Erfindung ist auch ein Verfahren zur Herstellung von Esterverbindungen der allgemeinen Formel I, 1 -2, mit R = OR', welches dadurch gekennzeichnet ist, dass ein Esterprodukt I ,1 -2, durch Umesterung mit einem Alkalialkoholat M'OR' (M' = Alkali) in ein entsprechendes Esterprodukt der Formel I, 1- 2 überführt wird, mit der Maßgabe, dass R' im eingesetzten Esterprodukt I, 1-2 nicht gleich dem Rest R' im eingesetzten Alkoholat M'OR' sein darf. Einmal hergestellte Ketomethioninketalester können auf diese Weise ganz leicht in andere gewünschte Ketomethioninketalester überführt werden.

Die nicht erfindungsgemäßen Ketomethioninketale der allgemeinen Formel I, 1 ,2 bzw. 5 mit R = OH oder OM bzw., X= H oder M sind polare und nicht lipophile Verbindungen. Aufgrund der Ketal-Schutzgruppe sind diese Verbindungen pansenstabil und können nicht mikrobiell abgebaut werden. Durch die lipophobe Carbonsäuregruppe werden sie jedoch nicht wie HMBi über die Pansenwand absorbiert, sondern gelangen ohne Abbau in den Labmangen (Abomasum) des Wiederkäuers, wo sie aufgrund der stark sauren Bedingungen hydrolysiert werden. Die Absorption des freiwerdenden Ketomethionins erfolgt dann anschließend im Dünndarm.

Bei den erfindungsgemäßen Ketomethioninketalestern der allgemeinen Formel I, 1, 2 mit R = OR' bzw. der allgemeinen Formel I, 4 handelt es sich um lipophile und nicht polare Verbindungen. Aufgrund der beiden chemischen Schutzgruppen "Ketal" und "Ester" sind diese Verbindungen pansenstabil. Die Absorption erfolgt im Gegensatz zu den Ketomethioninketalen schnell und effektiv über die Pansenwand analog dem Mechanismus von HMBi. Die anschließende enzymatische Spaltung zum freien Ketomethionin erfolgt dann im Blut des Wiederkäuers.

Die Verwendung von Ketomethioninketalen oder Ketomethioninketalester ermöglicht damit erstmals eine aktive Steuerung der Absorptionsstelle des "Methioninäquivalents".

Die Ketomethioninketale bzw. Ketomethioninketalester und deren Derivate haben mehrere Vorteile gegenüber den im Stand der Technik bekannten Verbindungen:
Im Gegensatz zum freien Ketomethionin sind
Ketomethioninketale, Ketomethioninketalester,
Ketomethioninhalbketalester und Ketomethioninketalamide der allgemeinen Formel I in Hinblick auf Dimerisierung und
Cyclisierung chemisch stabil, eine Voraussetzung für die Lagerung und Transport als Futtermitteladditive.

Die Verwendung der Verbindungen der allgemeinen Formel I erlaubt die aktive Steuerung der Absorptionsstelle im Organismus des Wiederkäuers. Dabei erfolgt die Absorption der hydrophilen Ketomethioninketale der Formel I, 1, 2 mit R = OH, OM oder der Formel I, 5 mit X = H, M im Dünndarm nach Hydrolyse im Abomasum bzw. die Absorption der lipophilen Ketomethioninketalester der allgemeinen Formel I, 1, 2 mit R = OR' bzw. der allgemeinen Formel I, 4 direkt über die Pansenwand.

Im Falle der Verwendung von bestimmten Diolen wie z.B. Ethylenglykol oder ausgewählten Triolen wie z.B. Glycerin oder Zuckern wie z.B. Glucose als Bausteine zur Ketal- bzw. Esterbildung mit Ketomethionin zu den entsprechenden Verbindungen der allgemeinen Formel I, besitzen diese zusätzlich eine nutritive Wirkung, aufgrund der im Organismus wieder freisetzbaren Zucker- bzw. Alkohol-Bausteine.

Ketomethioninketale und Ketomethioninketalester besitzen eine sehr hohe Biowertigkeit, da sie *in vivo* zu α-Ketomethionin hydrolysiert werden können. Die Biowertigkeit von Ketomethionin ist dabei wesentlich höher als die von MHA, da es im Gegensatz dazu in nur einer Stufe im Organismus zu L-Methionin umgewandelt werden kann. Dagegen benötigt MHA zwei und HMBi sogar drei Stufen.

Die hohe Biowertigkeit der Ketomethioninketale und Ketomethioninketalester stellt einen deutlichen wirtschaftlichen Vorteil dar, da weniger Futtermitteladditiv benötigt wird.

Aufgrund des chemischen Schutzes kann das Produkt und damit der Pansenschutz nicht durch physikalische Kräfte wie z.B. Reibung beschädigt werden. Daher ist es im Vergleich zu einer physikalisch geschützten Methioninform wie z.B. Smartamine möglich, Ketomethioninketale, Ketomethioninketalester, Ketomethioninhalbketalester und Ketomethioninketalamide der allgemeinen Formel I zu pelletieren. Dies stellt einen außerordentlichen Vorteil dar, weil damit eine breite Verwendbarkeit bei der Mischfutter-Herstellung und -Konfektionierung sichergestellt wird.

Darüber hinaus können die genannten Verbindungen der allgemeinen Formel I generell als Futtermitteladditive in der Nutztierhaltung eingesetzt werden, also auch bei der Ernährung von Geflügel oder Schweinen.

Insbesondere Ketomethioninketale und Ketomethioninketalester können auf einfachere Art und damit zu in der Regel niedrigeren Herstellkosten als physikalisch geschützte Methioninformen produziert werden.

Die symmetrischen Ketomethioninketale der allgemeinen Formel I sind achiral im Gegensatz zu MHA, HMBi oder D,L-Methionin. Das natürliche L-Methionin wird aus diesen achiralen Vorstufen im tierischen Organismus direkt gebildet. Eine Umwandlung des unnatürlichen Enantiomeren entfällt dadurch. Die nachfolgenden Beispiele zeigen Möglichkeiten zur Herstellung der erfindungsgemäßen Verbindungen auf ohne dabei beschränkend zu wirken.

### Beispiele:

### Beispiel 1: Freisetzung von Ketomethionin aus seinen Salzen (nicht erfindungsgemäß):

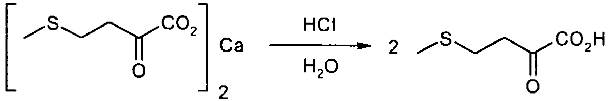

Zu einer Suspension aus 43.3 g Calcium-Ketomethioninat (M = 334.42 g/mol, 98% Reinheit, in der Trockenmasse 21% Wassergehalt) in 120 ml H₂O und 320 ml Diethylether wurde bei 0°C unter kräftigem Rühren eine 10%ige wässrige Salzsäurelösung langsam zugetropft bis der pH-Wert < 2 erreicht wurde. Nach der Phasentrennung wurde die wässrige Phase dreimal mit je 120 ml Diethylether gewaschen. Anschließend werden die vereinten organischen Phasen über Na₂SO₄ getrocknet. Nach der Filtration des Trockenmittels wurde der Diethylether am Rotationsverdampfer bei 30°C und leichtem Vakuum abdestilliert. Die letzten Lösungsmittelreste wurden im Hochvakuum entfernt. Zurück blieben 29.1 g eines leicht gelblichen Öls an freiem Ketomethionin (Ausbeute = 98%, M = 148.18 g/mol).

¹H-NMR von Calcium-Ketomethioninat 500 MHz, DMSO-d6):
δ = 2.04 (s, 3H, CH₃), 2.62 (t, ³*J* = 7.3 Hz, 2H, CH₂), 2.82 (t, ³*J* = 7.3 Hz, 2H, CH₂).
¹H-NMR von Ketomethionin (500 MHz, DMSO-d6):
   δ = 2.06 (s, 3H, CH₃), 2.66 (t, ³*J* = 7.2 Hz, 2H, CH₂), 3.09 (t, ³*J* = 7.2 Hz, 2H, CH₂).
   ¹³C-NMR von Ketomethionin (125.8 MHz, DMSO-d6): δ = 14.7 (CH₃), 26.7 (CH₂), 38.5 (CH₂), 162.2 (COOH), 194.9 (CO).

### Beispiel 2: Herstellung von Ketomethioninketalen der Formel I, 2 am Beispiel der Umsetzung von Ketomethionin und Ethylengykol:

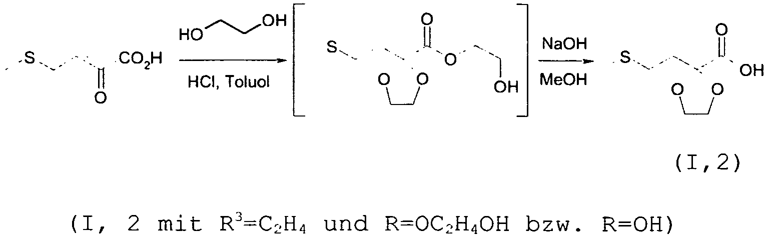

Eine Lösung von Ketomethionin (174 mmol, M = 148.18 g/mol) in Toluol (100 ml) wurde über 2 h tropfenweise zu einer Lösung von 1 g (~3 Mol%) *p*-Toluolsulfonsäure als Katalysator und Ethylenglykol (335 mmol, M = 62.07 g/mol) in Toluol (250 ml) zugegeben und die Reaktion solange unter Rückfluss gehalten bis sich am aufgesetzten Wasserabscheider kein weiteres Wasser mehr abschied (ca. 2 h). Anschließend wurde das Toluol unter Vakuum abgezogen und das Rohprodukt mit Methanol (200 ml) versetzt und nach Zugabe einer wässrigen Lösung von 2M Natriumhydroxid (200 ml) für ca. 2h alkalisch verseift. Die Reaktionslösung wurde anschließend mit Diethylether ausgeschüttelt und die wässerige Phase mit verdünnter Salzsäure angesäuert. Das Produkt wurde bei pH 1-2 mit Diethylether extrahiert, die organische Phase mit Wasser gewaschen, über Na₂SO₄ getrocknet und das Lösungsmittel am Rotationsverdampfer entfernt. Das ölige Produkt (I,2) wurde anschließend aus Methylenchlorid/n-Hexan umkristallisiert und fiel als weißer kristalliner Feststoff an. (24.6 g, Ausbeute = 74 %, M = 192,23 g/mol, Schmelzpunkt: 74°C Methylenchlorid/n-Hexan).

¹H-NMR von 2-(2-(Methylthio)ethyl)-1,3-dioxolan-2-carbonsäure (I,2) (500 MHz, CDCl₃) : δ = 2.11 (s, 3H, SCH₃), 2.24-2.28 (m, 2H, CH₂), 2.58-2.61 (m, 2H, CH₂), 4.07-4.14 (m, 4H, OCH₂CH₂O).

¹³C-NMR von 2-(2-(Methylthio)ethyl)-1,3-dioxolan-2-carbonsäure (I,2) (125.8 MHz, CDCl₃) : δ = 15.5 (SCH₃), 27.1 (CH₂), 34.9 (CH₂), 66.1 (2 OCH₂), 105.9 (C), 174.1 (COO).

Elementaranalyse für C₇H₁₂O₄S (M = 192,24 g/mol): C 43.74; H 6.29; S 16.68 Gefunden: C 43.80; H 6.25; S 16.61.

### Beispiel 3: Herstellung vom Calciumsalz des Ketomethioninketals durch Neutralisation (nicht Teil der Erfindung):

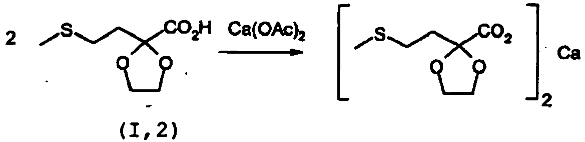

Zu einer Lösung aus 1.0 g (5.20 mmol) 4-(Methylthio)-2-ketobuttersäure-ethylenketal in 2.0 ml Wasser und 3.0 ml Aceton wurde bei RT langsam eine wässrige Lösung aus 0.44 g Calciumacetat (93%ig) in 2 ml Wasser zugetropft. Anschließend wurden 3 ml Aceton zugegeben und über Nacht bei RT gerührt. Der gebildete weiße Feststoff wurde abgesaugt und mit 100 ml einer 1:10 Wasser-Aceton-Mischung gründlich gewaschen. Das Produkt wurde anschließend im Trockenschrank bzw. im Hochvakuum getrocknet. (0.96 g, Ausbeute = 82%, 6.3% Wassergehalt nach K.F.-Methode).

¹H-NMR von Calcium 2-(2-(Methylthio)ethyl)-1,3-dioxolan-2-carboxylat (1,2) (500 MHz, DMSO-D6): δ = 1.98-2.00 (m, 2H, CH₂, 2.03 (s, 3H, SCH₃), 2.44-2.47 (m, 2H, CH₂), 3.81-3.84 (m, 2H, CH₂), 3.95-4.00 (m, 2H, CH₂).

¹³C-NMR von Calcium 2-(2-(Methylthio)ethyl)-1,3-dioxolan-2-carboxylat (1,2) (125.8 MHz, DMSO-D6/DCl): δ = 15.1 (SCH₃), 27.0 (CH₂), 35.2 (CH₂), 65.7, 66.0 (2 OCH₂), 105.6 (C), 171.0 (COO).

### Beispiel 4: Herstellung von Ketomethioninhalbketalestern I, 3 am Beispiel der Umsetzung von Ketomethionin und 2,2-Dimethyl-1,3-propandiol:

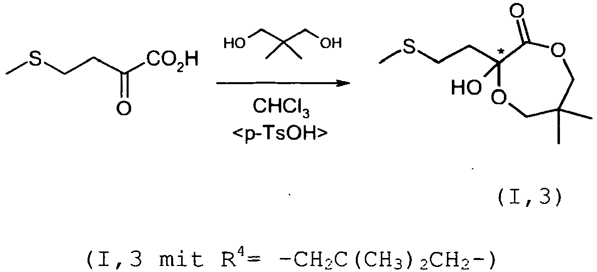

Zu einer Lösung aus 11.1 g Ketomethionin (75 mmol, M=148.18 g/mol) in 200 ml absolutem ethanolfreiem Chloroform wurden 8.9 g 2,2-Dimethyl-1,3-propandiol (85 mmol, M=104.15 g/mol) und 0.8 g(~5 Mol%) p-Toluolsulfonsäure als Katalysator zugegeben und solange unter Rückfluss gehalten bis sich am aufgesetzten Wasserabscheider.kein weiteres Wasser mehr abschied (ca. 3.5 h). Nach dem Abkühlen wurde die Lösung mit halbgesättigter NaHCO₃-Lösung gewaschen, die wässrige Phase nochmals mit Chloroform rückgewaschen und die vereinten organischen Phasen über Na₂SO₄ getrocknet. Nach Filtration wurde das Lösungsmittel am Rotationsverdampfer entfernt und das ölige Rohprodukt aus Methylenchlorid/*n-*Hexan kristallisiert. Das Produkt (I,3) fiel als weißer kristalliner Feststoff an (10.8 g, Ausbeute = 62%, M = 234,32 g/mol, Schmelzpunkt = 109°C (Methylenchlorid/*n-*Hexan)).

¹H-NMR von rac-4,4-Dimethyl-7-hydroxy-7-(2-(methylthio)-ethyl)-6-oxa-caprolacton (I,3) (500 MHz, CDCl₃) : δ = 0.92 (s, 3H, CH₃), 0.99 (s, 3H, CH₃), 2.04-2.19 (m, 2H, CH₂), 2.08 (s, 3H, SCH₃),2.47-2.52 (m, 1H, SCHH), 2.61-2.66 (m, 1H, SCHH), 2.92 (d, ²*J* = 8.0 Hz, 1H, OCHH), 3.25 (d, ²*J* = 8.0 Hz, 1H, OCHH), 3.72 (d, ²*J* = 10.4 Hz, 1H, COOCHH), 4.30 (d, ²*J* = 10.4 Hz, 1H, COOCHH), 4.50 (s, 1H, OH).

¹³C-NMR von rac-4,4-Dimethyl-7-hydroxy-7-(2-(methylthio)-ethyl)-6-oxa-caprolacton (I,3) (125.8 MHz, CDCl₃) : δ = 15.7 (SCH₃), 21.9, 21.3 (2 CH₃), 27.7 (CH₂), 37.7 (CH₂), 66.0 (OCH₂), 70.9 (OCH₂), 96.5 (COH), 172.5 (COO).

Elementaranalyse für C₁₀H₁₈O₄S (M = 234,32 g/mol): C 51.26; H 7.74; S 13.68 Gefunden: C 50.82; H 7.73; S 13.52.

### Beispiel 5: Herstellung von Ketomethioninketalestern I,4 am Beispiel der Umsetzung von Ketomethionin und Glycerin:

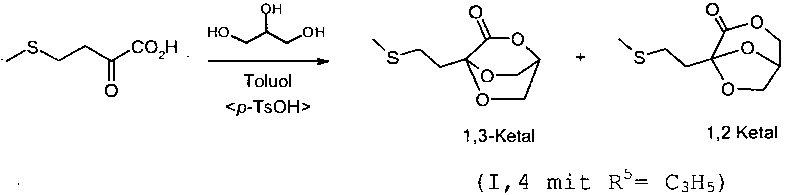

Zu einer Lösung aus 12.0 g Keto-Methionin (81 mmol, M = 148.18 g/mol) in 120 ml absolutem Toluol wurden 7.8 g Glycerin (1,2,3-Propantriol)(85 mmol, M = 92.09 g/mol) und 0.8 g (~5 Mol%) p-Toluolsulfonsäure als Katalysator zugegeben und solange unter Rückfluss gehalten bis sich am aufgesetzten Wasserabscheider kein weiteres Wasser mehr abschied (ca. 2.5 h). Nach dem Abkühlen wurde die Lösung mit halbgesättigter NaHCO₃-Lösung gewaschen, die wässrige Phase nochmals mit Chloroform rückgewaschen und die vereinten organischen Phasen über Na₂SO₄ getrocknet. Nach Filtration wurde das Lösungsmittel am Rotationsverdampfer entfernt und das ölige Rohprodukt (Verhältnis (1,3-Ketal: 1,2-Ketal = 70:30) chromatographiert (Diethylether/n-Hexan 1:1). Dabei wurden die beiden Verbindungen voneinander getrennt. Das Hauptprodukt (1,3-Ketal) kristallisierte in Form farbloser Nadeln aus einer Mischung aus Methylenchlorid/n-Hexan aus (8.8 g, Ausbeute = 53%, M = 204,25 g/mol, Schmelzpunkt = 39.5°C (Methylenchlorid/n-Hexan)).

¹H-NMR von 4-(2-(Methylthio)ethyl)-2,5,8-trioxabicyclo[2.2.2]-octan-3-on (1,3-Ketal) (500 MHz, CDCl₃): δ = 2.13 (s, 3H, SCH₃), 2.17-2.20 (m, 2H, CH₂), 2.65-2.68 (m, 2H, CH₂), 4.12-4.13 (m, 4H, 2 CH₂), 4.76 (s, 1H, CH).

¹³C-NMR von 4-(2-(Methylthio)ethyl)-2,5,8-trioxabicyclo[2.2.2]-octan-3-on (1,3-Ketal) (125.8 MHz, CDC1₃) : δ = 15.4 (SCH₃), 26.9 (CH₂), 33.2 (CH₂), 66.5 (2 OCH₂), 70.9 (CH), 92.9 (C), 166.2 (COO).

Elementaranalyse für C₈H₁₂O₄S (M = 204,25 g/mol): C 47.04; H 5.92; S 15.70 Gefunden: C 47.21; H 5.93; S 15.69.

### Beispiel 6: Herstellung von Ketomethioninketalsalzen der Formel I, 5 am Beispiel der Verseifung von 1,2-Ketal bzw. 1,3-Ketal aus Beispiel 5 (nicht Teil der Erfindung);

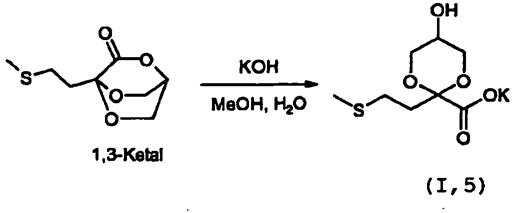

Zu einer Suspension aus 50 mg (0.24 mmol) des 1,3-Ketals aus Beispiel 5 in 1.0 ml Methanol und 1.5 ml Wasser wurde bei RT 15 mg Kaliumhydroxid zugegeben und die Lösung 30 Min bei RT gerührt. Nach Entfernen des Lösungsmittels und Trocknung unter Vakuum wurde das Produkt als ein weißer Feststoff erhalten. (0.60 g, Ausbeute = 96%).

¹H-NMR von Kalium 5-hydroxy-2-(2-(methylthio)ethyl)-1,3-dioxan-2-carboxylat (500 MHz, D₂O/TSP): δ = 1.96-2.02 (m, 2H, CH₂), 2.10 (t, 3H, CH₃), 2.51-2.54 (m, 2H, CH₂), 3.48-3.52 (m, 2H, CH₂), 3.81-3.87 (m, 1H, CH), 3.98-4.01 (m, 2H, CH₂).

## Patentansprüche

1. Chemische Verbindung der allgemeinen Formel I 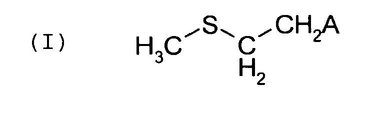 wobei A = 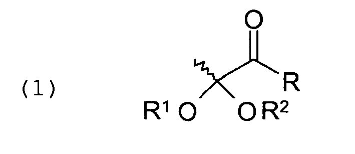 und R = OR', ist, wobei R¹, R² und R' gleich oder verschieden ist und jeweils ein verzweigtes oder geradkettiges C₁-C₁₈-Alkyl- bzw.
C₃-C₁₈-Cycloalkyl-,
Allyl-,
Benzyl-,
Phenyl- oder
C₁-C₁₈-Alkyloxymethyl-, vorzugsweise C₂H₅OCH₂ C₂-C₆-Hydroxyalkyl-, vorzugsweise HOC₂H₄-, C₃-C₆-Dihydroxyalkyl-, vorzugsweise (HO)₂C₃H₅-, oder ein C₃-C₁₂-Zuckerrest, bei dem eine OH-Gruppe des Zuckers jeweils vom Ketal-O-Atom oder vom Carbonsäure-O-Atom ersetzt ist,
oder A = 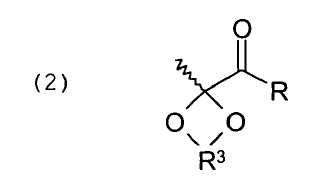 und R = OR' ist,
wobei R' jeweils ein verzweigtes oder geradkettiges C₁-C₁₈-Alkyl- bzw.
C₃-C₁₈-Cycloalkyl-,
Allyl-,
Benzyl-,
Phenyl- oder
C₁-C₁₈-Alkyloxymethyl-, vorzugsweise C₂H₅OCH₂-,
C₂-C₆-Hydroxyalkyl-, vorzugsweise HOC₂H₄-,
C₃-C₆-Dihydroxyalkyl-, vorzugsweise (HO)₂C₃H₅-, oder ein C₃-C₁₂-Zuckerrest, bei dem eine OH-Gruppe des Zuckers vom Carbonsäure-O-Atom ersetzt ist
und R³ eine C₂-C₄-Alkylenbrücke darstellt, vorzugsweise C₂H₄, oder einen C₃-C₁₂-Zuckerrest, bei dem zwei OH-Gruppen des Zuckers von den beiden Ketal-O-Atomen ersetzt sind,
oder A = 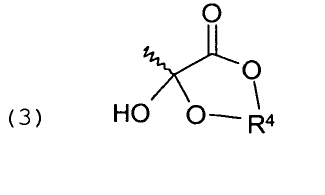 und R⁴ ein C₃-C₆-Alkylenrest, der ggf. Hydroxysubstituiert ist, vorzugsweise -CH₂-C(CH₃)₂-CH₂- und -CH₂-C(CH₂OH)₂-CH₂- ist, oder ein C₃-C₁₂-Zuckerrest, bei dem eine OH-Gruppe des Zuckers vom Ketal-O-Atom und eine vom Carbonsäure-O-Atom ersetzt ist,
oder A = 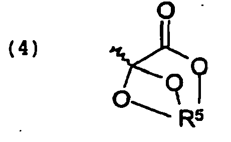 und R⁵ ein C₃-C₆-Alkylenrest, der ggf. hydroxysubstituiert ist, vorzugsweise -CH₂-CH(-)-CH₂-, ist, oder ein C₃-C₁₂-Zuckerrest, bei dem drei OH-Gruppen des Zuckers von beiden Ketal-O-Atomen und vom Carbonsäure-O-Atom ersetzt sind.

2. Verbindung gemäß Anspruch 1, **dadurch gekennzeichnet,**
**dass** R = OR' und R'= C₁-C₁₈-Alkyl und R³ = C₂H₄ ist, bevorzugt
**dass** R' = C₁-C₄-Alkyl ist, oder
**dass** R = OR' und R'= Hydroxyethoxy und R³ = C₂H₄ ist, oder dass R⁴ ein C₅H₁₀-Rest oder ein C₅H₁₀O₂-Rest ist.

3. Verbindung gemäß Anspruch 2, **gekennzeichnet durch** die Formel:

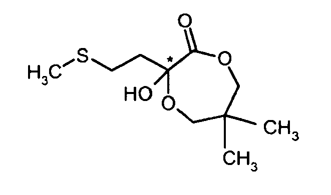

4. Verbindung gemäß Anspruch 1, **dadurch gekennzeichnet, dass** R⁵ ein C₃H₅-Rest ist.

5. Verbindung gemäß Anspruch 4, **gekennzeichnet durch** die Formel: 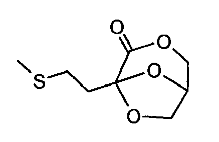 oder
**durch** die Formel:

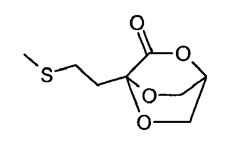

6. Verwendung von Verbindungen gemäß Anspruch 1 - 5 in der Tierernährung.

7. Verwendung gemäß Anspruch 6 zur Ernährung von Wiederkäuern.

8. Verwendung von Verbindungen gemäß Anspruch 1 - 5 zur Herstellung von Futtermitteln.

9. verwendung gemäß Anspruch 8 zur Herstellung von Futtermitteln für Wiederkäuer.

10. Futtermittelzubereitung, enthaltend mindestens eine Verbindung gemäß Anspruch 1 - 5.

11. Futtermittelzubereitung gemäß Anspruch 9 zur Ernährung von Wiederkäuern.

12. Verfahren zur Herstellung von Carbonsäure- oder Esterverbindungen der allgemeinen Formel I, 1 - 4 gemäß Anspruch 1, **dadurch gekennzeichnet, dass** man Ketomethionin mit einem entsprechenden einwertigen, zweiwertigen, dreiwertigen Alkohol oder einem C₃-C₁₂-Zucker in Gegenwart saurer Katalysatoren zum Esterprodukt der Formel 1 - 2, mit R = OR' oder zum Esterprodukt 3-4
umsetzt.

13. Verfahren gemäß Anspruch 12, **dadurch gekennzeichnet, dass** als Alkohol ein verzweigter oder geradekettiger C₁-C₁₈-Alkylalkohol bzw. C₃-C₁₈-Cycloalkylalkohol, Allylalkohol, Benzylalkohol, Phenylalkohol, C₂-C₆-Hydroxyalkylalkohol, vorzugsweise HOC₂H₄OH, C₃-C₆-Dihydroxyalkylalkohol, vorzugsweise Glycerin, oder einen C₃-C₁₂-Zucker, vorzugsweise Glycerinaldehyd, Dihydroxyaceton, Glucose, Fructose oder Saccharose eingesetzt werden.

14. Verfahren gemäß einem der Ansprüche 12 - 13, **dadurch gekennzeichnet, dass** während der Reaktion entstehendes Wasser entfernt wird.

15. Verfahren zur Herstellung von Esterverbindungen der allgemeinen Formel I, 1 -2 gemäß Anspruch 1, mit R = OR', **dadurch gekennzeichnet, dass** ein Esterprodukt I,1 -2, durch Umesterung mit Alkalialkoholat M'OR' in ein entsprechendes Esterprodukt der Formel I, 1 - 2 überführt wird, mit der Maßgabe, dass R' im eingesetzten Esterprodukt 1-2 nicht gleich dem Rest R' im eingesetzten Alkoholat M'OR' sein darf.

## Claims

1. Chemical compound of the general formula I 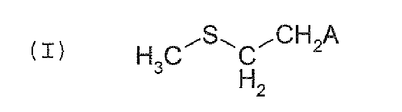 wherein A = 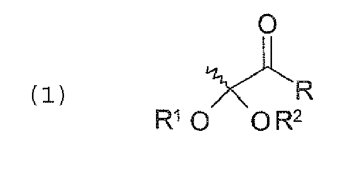 and R = OR', wherein R¹, R² and R' are identical or different and are in each case a branched or straight-chain C₁-C₁₈-alkyl or
C₃-C₁₈-cycloalkyl,
allyl,
benzyl,
phenyl or
C₁-C₁₈-alkyloxymethyl, preferably C₂H₅OCH₂ C₂-C₆-hydroxyalkyl, preferably HOC₂H₄-,
C₃-C₆-dihydroxyalkyl, preferably (HO)₂C₃H₅-, or a C₃-C₁₂ sugar radical in which one OH group of the sugar is replaced in each case by the ketal-O atom or by the carboxylic acid-O atom,
or A = 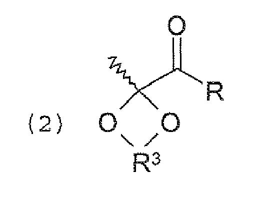 and R = OR',
wherein R' is in each case a branched or straight-chain C₁-C₁₈-alkyl or
C₃-C₁₈-cycloalkyl,
allyl,
benzyl,
phenyl or
C₁-C₁₈-alkyloxymethyl, preferably C₂H₅OCH₂
C₂-C₆-hydroxyalkyl, preferably HOC₂H₄-,
C₃-C₆-dihydroxyalkyl, preferably (HO)₂C₃H₅-, or a C₃-C₁₂ sugar radical in which one OH group of the sugar is replaced by the carboxylic acid-O atom,
and R³ is a C₂-C₄-alkylene bridge, preferably C₂H₄, or a C₃-C₁₂ sugar radical in which two OH groups of the sugar are replaced by the two ketal-O atoms,
or A = 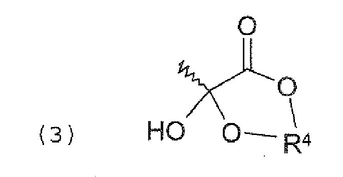 and R⁴ is a C₃-C₆-alkylene radical which is optionally hydroxy substituted, preferably -CH₂-C(CH₃)₂-CH₂- and -CH₂-C (CH₂OH)₂-CH₂-, or a C₃-C₁₂ sugar radical in which one OH group of the sugar is replaced by the ketal-O atom and one is replaced by the carboxylic acid-O atom,
or A = 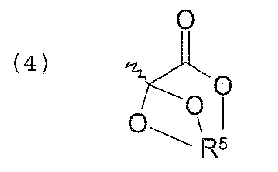 and R⁵ is a C₃-C₆-alkylene radical which is optionally hydroxy substituted, preferably -CH₂-CH(-)-CH₂-, or a C₃-C₁₂ sugar radical in which three OH groups of the sugar are replaced by both ketal-O atoms and by the carboxylic acid-O atom.

2. Compound according to Claim 1, **characterized in that**
R = OR' and R' = C₁-C₁₈-alkyl and R³ = C₂H₄, preferably
**in that** R' = C₁-C₄-alkyl, or
**in that** R = OR' and R' = hydroxyethoxy and R³ = C₂H₄, or
**in that** R⁴ is a C₅H₁₀ radical or a C₅H₁₀O₂ radical.

3. Compound according to Claim 2, **characterized by** the formula:

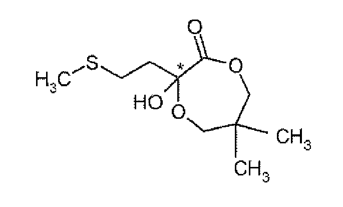

4. Compound according to Claim 1, **characterized in that** R⁵ is a C₃H₅ radical.

5. Compound according to Claim 4, **characterized by** the formula: 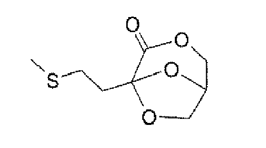 Or
by the formula:

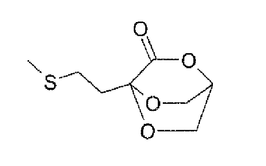

6. Use of compounds according to Claim 1-5 in animal nutrition.

7. Use according to Claim 6 for the nutrition of ruminants.

8. Use of compounds according to Claim 1-5 for producing feeds.

9. Use according to Claim 8 for producing feeds for ruminants.

10. Feed preparation containing at least one compound according to Claim 1-5.

11. Feed preparation according to Claim 9 for nutrition of ruminants.

12. Process for producing carboxylic acid or ester compounds of the general formula I, 1-4 according to Claim 1, **characterized in that** ketomethionine is reacted with a corresponding monovalent, divalent, trivalent alcohol or a C₃-C₁₂ sugar in the presence of acid catalysts to give the ester product of the formulae 1-2, where R = OR', or to give the ester product 3-4.

13. Process according to Claim 12, **characterized in that**, as alcohol, use is made of a branched or straight-chain C₁-C₁₈-alkyl alcohol or C₃-C₁₈-cycloalkyl alcohol, allyl alcohol, benzyl alcohol, phenyl alcohol, C₂-C₆-hydroxyalkyl alcohol, preferably HOC₂H₄OH, C₃-C₆-dihydroxyalkyl alcohol, preferably glycerol, or a C₃-C₁₂ sugar, preferably glyceraldehyde, dihydroxyacetone, glucose, fructose or sucrose.

14. Process according to one of Claims 12-13, **characterized in that** water formed during the reaction is removed.

15. Process for producing ester compounds of the general formula I, 1-2 according to Claim 1, where R = OR', **characterized in that** an ester product I, 1-2, is converted into a corresponding ester product of the formula I, 1-2 by transesterification with alkali metal alkoxide M'OR', with the proviso that R' in the ester product 1-2 used must not be equal to the radical R' in the alkoxide M'OR' used.

## Revendications

1. Composé chimique de formule générale I 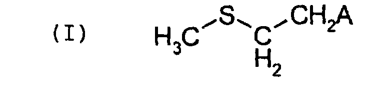 où A = 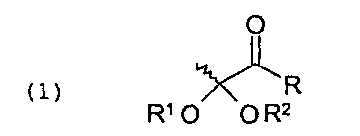 et R = OR' , R¹, R² et R' étant identiques ou différents et représentant à chaque fois un radical C₁-C₁₈-alkyle linéaire ou ramifié ou, selon le cas
un radical C₃-C₁₈-cycloalkyle,
un radical allyle,
un radical benzyle,
un radical phényle ou
un radical C₁-C₁₈-alkyloxyméthyle, de préférence C₂H₅OCH₂ un radical C₂-C₆-hydroxyalkyle, de préférence HOC₂H₄,
un radical C₃-C₆-dihydroxyalkyle, de préférence (HO)₂C₃H₅, ou un radical C₃-C₁₂-sucre, dans lequel un groupe OH du sucre est remplacé à chaque fois par l'atome d'O du cétal ou par l'atome d'O de l'acide carboxylique,
ou A = 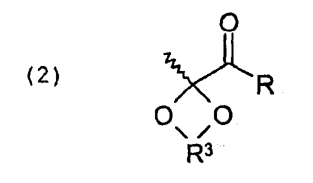 et R = OR',
R' représentant à chaque fois un radical C₁-C₁₈-alkyle linéaire ou ramifié ou, selon le cas
un radical C₃-C₁₈-cycloalkyle,
un radical allyle,
un radical benzyle,
un radical phényle ou
un radical C₁-C₁₈-alkyloxyméthyle, de préférence C₂H₅OCH₂,
un radical C₂-C₆-hydroxyalkyle, de préférence HOC₂H₄,
un radical C₃-C₆-dihydroxyalkyle, de préférence (HO)₂C₃H₅, ou un radical C₃-C₁₂-sucre, dans lequel un groupe OH du sucre est remplacé par l'atome d'O de l'acide carboxylique,
et R³ représente un pont C₂-C₄-alkylène, de préférence C₂H₄, ou un radical C₃-C₁₂-sucre, dans lequel deux groupes OH du sucre sont remplacés par les deux atomes d'O du cétal,
ou A = 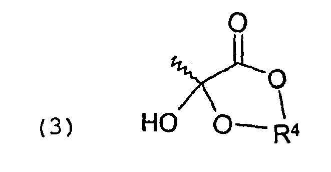 et R⁴ représente un radical C₃-C₆-alkylène, qui est le cas échéant substitué par hydroxy, de préférence -CH₂-C(CH₃)₂-CH₂- et -CH₂-C(CH₂OH)₂-CH₂-, ou un radical C₃-C₁₂-sucre, dans lequel un groupe OH du sucre est remplacé par l'atome d'O du cétal et un par l'atome d'O de l'acide carboxylique,
ou A = 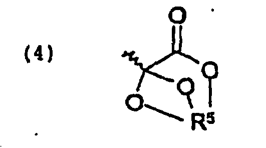 et R⁵ représente un radical C₃-C₆-alkylène, qui est le cas échéant substitué par hydroxy, de préférence -CH₂-CH(-)-CH₂-, ou un radical C₃-C₁₂-sucre, dans lequel les trois groupes OH du sucre sont remplacés par les deux atomes d'O du cétal et par l'atome d'O de l'acide carboxylique.

2. Composé selon la revendication 1, **caractérisé en ce que** R = OR' et R' = C₁-C₁₈-alkyle et R³ = C₂H₄, de préférence **en ce que** R' = C₁-C₄-alkyle, ou **en ce que** R = OR' et R' = hydroxyéthoxy et R³ = C₂H₄, ou **en ce que** R⁴ représente un radical C₅H₁₀ ou un radical C₅H₁₀O₂.

3. Composé selon la revendication 2, **caractérisé par** la formule :

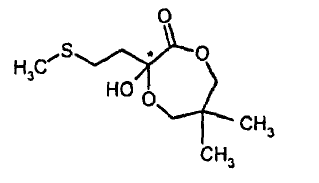

4. Composé selon la revendication 1, **caractérisé en ce que** R⁵ représente un radical C₃H₅.

5. Composé selon la revendication 4, **caractérisé par** la formule : 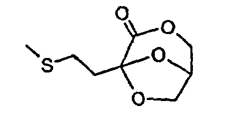 ou
par la formule :

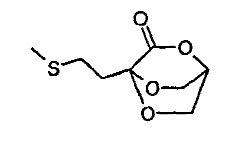

6. Utilisation de composés selon la revendication 1-5 dans l'alimentation animale.

7. Utilisation selon la revendication 6 pour l'alimentation de ruminants.

8. Utilisation des composés selon la revendication 1-5 pour la préparation de fourrages.

9. Utilisation selon la revendication 8 pour la préparation de fourrages pour ruminants.

10. Préparation fourragère, contenant au moins un composé selon la revendication 1-5.

11. Préparation fourragère selon la revendication 9 pour l'alimentation de ruminants.

12. Procédé pour la préparation de composés d'acide carboxylique ou d'ester de formule générale I, 1-4 selon la revendication 1, **caractérisé en ce qu'**on transforme de la cétométhionine avec un alcool monovalent, divalent ou trivalent correspondant ou un C₃-C₁₂-sucre en présence de catalyseurs acides en produit d'ester de formule 1-2, R = OR' ou en produit d'ester 3-4.

13. Procédé selon la revendication 12, **caractérisé en ce qu'**on utilise comme alcool un alcool C₁-C₁₈-alkylique ramifié ou linéaire ou, selon le cas, un alcool C₃-C₁₈-cycloalkylique, l'alcool allylique, l'alcool benzylique, l'alcool phénylique, un alcool C₂-C₆-hydroxyalkylique, de préférence HOC₂H₄OH, un alcool C₃-C₆-dihydroxyalkylique, de préférence le glycérol, ou un C₃-C₁₂-sucre, de préférence le glycérolaldéhyde, la dihydroxyacétone, le glucose, le fructose ou le saccharose.

14. Procédé selon l'une quelconque des revendications 12-13, **caractérisé en ce que** l'eau formée pendant la réaction est éliminée.

15. Procédé pour la préparation de composés d'ester de formule générale I, 1-2 selon la revendication 1, avec R = OR', **caractérisé en ce qu'**un produit d'ester I, 1-2, est transformé, par transestérification avec un alcoolate de métal alcalin M'OR', en un produit d'ester correspondant de formule I, 1-2, à condition que R' dans le produit d'ester utilisé 1-2 ne soit pas identique au radical R' dans l'alcoolate M'OR' utilisé.
